# EUROPÄISCHE PATENTSCHRIFT

(11) **EP 0 784 666 B1**
(45) Veröffentlichungstag und Bekanntmachung des Hinweises auf die Patenterteilung: **12.05.1999**
(21) Anmeldenummer: 95934645.3
(22) Anmeldetag: 25.09.1995
(51) Int. Cl.: C11D 3/37, C11D 1/62, D06M 15/227, D06M 13/463, A61K 7/50

(54) **WÄSSRIGE WEICHSPÜLERDISPERSIONEN**
AQUEOUS SOFT RINSING DISPERSIONS
DISPERSIONS AQUEUSES D'AGENTS ASSOUPLISSANTS

(30) Priorität: 04.10.1994 DE 4435386
(43) Veröffentlichungstag der Anmeldung: 23.07.1997
(73) Patentinhaber: Henkel Kommanditgesellschaft auf Aktien, 40191 Düsseldorf (DE)
(72) Erfinder: BONASTRE, Nuria, E-080210 Barberà des Vallès (ES); PI-SUBIRANA, Raffael, E-08400 Granollers (ES); CLOSA CRUXENS, Xavier, E-08190 St. Cugat des Vallès (ES)
(86) Internationale Anmeldenummer: EP9503793
(87) Internationale Veröffentlichungsnummer: WO9610623

(56) Entgegenhaltungen:
- EP-A- 0 374 609
- EP-A- 0 416 917
- WO-A-93/16157
- DD-A- 234 687
- DE-A- 2 165 947

## Beschreibung

### Gebiet der Erfindung

Die Erfindung betrifft wäßrige Weichspülerdispersionen mit einem Gehalt an Esterquats und Polyolefinwachsen, ein Verfahren zu ihrer Herstellung sowie die Verwendung von Polyolefinwachsen zur Verdickung von wäßrigen Esterquatlösungen.

### Stand der Technik

Kationische Tenside vom Typ der sogenannten "Esterquats" haben in den letzten Jahren als ökotoxikologisch unbedenkliche Rohstoffe für Wäscheweichspüler zunehmend an Bedeutung gewonnen. Üblicherweise gelangen die Produkte als alkoholische Konzentrate in den Verkehr und werden von den Herstellem von Avivagemitteln mit Wasser auf eine Anwenderkonzentration, die je nach Anforderung im Bereich von 1 bis 50 Gew.-% Feststoff liegen kann, verdünnt. Sowohl bei der Herstellung von Esterquatkonzentraten, als auch bei der Formulierung der wäßrigen Mittel, die diese kationischen Tenside in unterschiedlichen Mengen enthalten können, treten Viskositätsprobleme auf. Esterquatkonzentrate mit einem Feststoffgehalt von etwa 80 bis 90 Gew.-% sind zähflüssig und benötigen als Regulatoren Stoffe, die die Viskosität herabsetzen. In der **EP-A2 0165138** wird hierzu beispielsweise die Verwendung von organischen Salzen wie etwa Natriumgluconat oder kurzkettigen quartären Ammoniumsalzen vorgeschlagen. Gemäß der Lehre der **WO 93/16157** (Henkel) kommen für diesen Zweck auch Esterquats auf Basis ungesättigter Fettsäuren, quaternierte Fettsäureamidoaminsalze, langkettige QAV und diquaternierte Fettsäuretriethanolaminestersalze in Betracht. Bei wäßrigen Anwendungslösungen der Esterquats, die einen geringen Feststoffgehalt aufweisen, liegt das Problem jedoch genau umgekehrt: die wäßrigen Lösungen sind äußerst dünnflüssig und lassen sich nur schwer dosieren. Übliche Verdickungsmittel, wie sie aus der Kosmetik bekannt sind (Elektrolyte, Celluloseether, Fettsäurealkanolamide etc.), erweisen sich entweder als wenig geeignet und/oder sind nicht in der Lage, die Produkte dauerhaft auf dem gewünschten Viskositätsniveau zu halten.

In diesem Zusammenhang sei auf die Druckschriften **EP-A 0374609, EP-A0416917** und **DD-A 234687** verwiesen, die wäßrige Weichmacherdispersionen aus Polyethylenwachsen und quartären Ammoniumverbindungen mit Estergruppen offenbaren.

Die Aufgabe der Erfindung hat daher darin bestanden, wäßrige Weichspülmittel auf Basis von Esterquats zur Verfügung zu stellen, die auch nach längerer Lagerung eine ausreichend hohe Viskosität aufweisen.

### Beschreibung der Erfindung

Gegenstand der Erfindung sind wäßrige Weichspüldispersion auf Basis kationischer Tenside und polymerer Wachse, die man erhält, indem man
(a) Polyolefinwachse in Wasser einrührt,
(b) Esterquats vom Typ der quaternierten Fettsäurealkanolaminestersalze sowie gegebenenfalls weitere Tenside und Hilfs- undloder Zusatzstoffe zugibt und
(c) die Mischung auf Anwendungskonzentration verdünnt.

Überraschenderweise wurde gefunden, daß der Zusatz von Polyolefinwachsen zu wäßrigen Esterquatlösungen eine vorteilhafte und dauerhafte Zunahme der Viskosität bewirkt.

### Verfahren

Ein weiterer Gegenstand der Erfindung betrifft ein Verfahren zur Herstellung von wäßrigen Weichspülerdispersionen, bei dem man
(a) Polyolefinwachse in Wasser einrührt,
(b) Esterquats vom Typ der quaternierten Fettsäurealkanolaminestersalze sowie gegebenenfalls weitere Tenside und Hilfs- undloder Zusatzstoffe zugibt und
(c) die Mischung auf die Anwendungskonzentration verdünnt.

In einer bevorzugten Ausführungsform der Erfindung werden etwa 40 bis 60, vorzugsweise etwa 50 Gew.-% des Wassers - bezogen auf das wäßrige Endprodukt - bei 20 bis 40°C vorgelegt und mit dem Polyolefinwachs, das in der Regel als wäßrige Dispersion vorliegt, unter Rühren versetzt. Anschließend werden die Esterquats sowie gegebenenfalls weitere Inhaltsstoffe eingerührt und schließlich mit der restlichen Menge Wasser verdünnt.

### Esterquats

Unter der Bezeichnung Esterquats werden quaternierte Fettsäurealkanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschiften **US 3915867, US 4370272, EP-A2 0239910, EP-A2 0293955, EP-A2 0295739** und **EP-A2 0309052** verwiesen. Die quaternierten Fettsäuretriethanolaminestersalze folgen der Formel **(I)**, 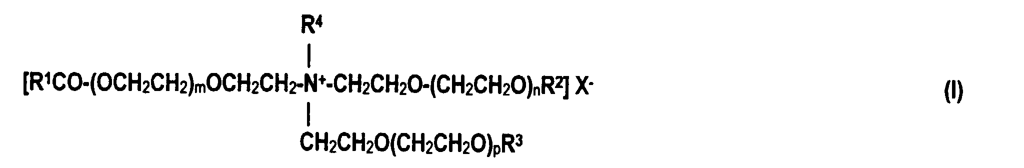 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(II)** in Betracht, 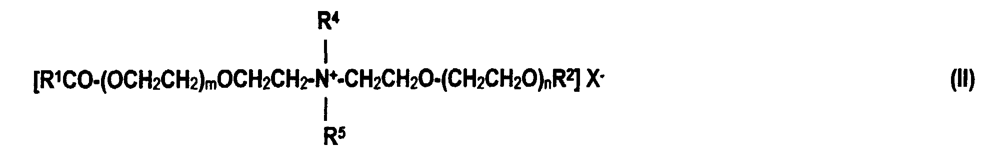 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als weitere Gruppe geeigneter Esterquats sind schließlich die quaternierten Estersalze von Fettsäuren mit 1,2-Dihydroxypropyldialkylaminen der Formel **(III)** zu nennen, 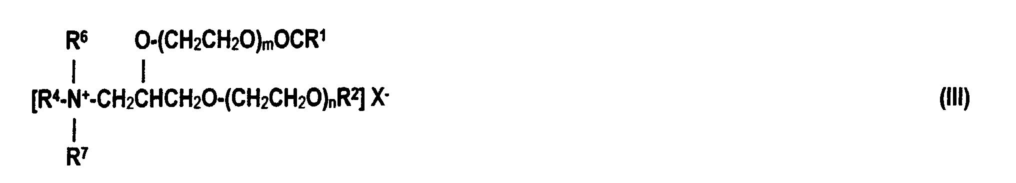 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können.

### Polyolefinwachse

Unter Polyolefinwachsen sind überwiegend lineare Polyolefine mit wachsartigem Charakter zu verstehen. Technische Bedeutung haben Polypropylen- und insbesondere Polyethylenwachse, bei denen es sich um Produkte mit relativ niedrigen Molmassen im Bereich von 500 bis 20.000 handelt. Die Herstellung der Polyolefinwachse erfolgt in der Regel durch direkte Hoch- oder Niederdruckpolymerisation der Monomere oder durch gezielte Depolymerisation von Produkten höherer Molmassen. Modifizierte Polyolefinwachse können durch Copolymerisation von Ethylen mit geeigneten anderen Monomeren wie beispielsweise Vinylacetat oder Acrylsäure hergestellt werden. Schließlich ist es möglich, die Dispergierbarkeit von Polyolefinen durch oxidative Oberflächenbehandlung zu verbessern. Übersichten zu diesem Thema finden sich beispielsweise in **Ullmann's Enzyklopaedie der technischen Chemie (4. Aufl.), 24, 36 sowie Enzcycl.Polym.Sci.Engng. 17, 792f.** Im Sinne des erfindungsgemäßen Verfahrens ist der Einsatz von high-density Polyethylenwachs mit einem mittleren Molekulargewicht im Bereich von 500 bis 10.000 und insbesondere 2.000 bis 5.000 bevorzugt. Vorzugsweise werden kationische bzw. pseudokationische Polyethylenwachse eingesetzt. Üblicherweise gelangen die Polyolefinwachse als wäßrige Dispersionen in den Handel und weisen einen Feststoffgehalt von 1 bis 25 und vorzugsweise 2 bis 15 Gew.-% auf. Üblicherweise werden die Esterquats und Polyolefinwachse - jeweils bezogen auf ihren Feststoffanteil - im Gewichtsverhältnis von 90 : 10 bis 50 : 50, vorzugsweise 80 : 20 bis 60 : 20 eingesetzt. Die wäßrigen Lösungen enthaltend die Esterquats, Polyolefinwachse und gegebenenfalls weitere Tenside, Hilfs- und Zusatzstoffe werden vorzugsweise auf eine Anwendungskonzentration von 1 bis 10, vorzugsweise 4 bis 8 Gew.-% Feststoff verdünnt.

### Tenside

Neben den Esterquats und den Polyolefinwachsen können die erfindungsgemäßen Mittel ferner weitere anionische, kationische, nichtionische und/oder amphotere bzw. zwitterionische Tenside enthalten. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acylglutamate, Acyltartrate, Alkyloligoglucosidsulfate und Alkyl(ether)phosphate. Sofem die anionischen Tenside Polyglycoletherketten enthalten, können sie eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Mischether, Alk(en)yloligoglykoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für weitere **kationische Tenside** sind quartäre Ammoniumverbindungen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Alkyl- und/oder Alkenylsulfate

In einer bevorzugten Ausführungsform der Erfindung können die Mittel anionische Tenside vom Typ der Alkyl- und/oder Alkenylsulfate enthalten. Unter diesen Aniontensiden sind die Sulfatierungsprodukte primärer Alkohole zu verstehen, die der Formel **(IV)** folgen,

**R**^{**8**}**O-SO**_{**3**}**Y** (IV)

in der R⁸ für einen linearen oder verzweigten, aliphatischen Alkyl- und/oder Alkenylrest mit 6 bis 22, vorzugsweise 12 bis 18 Kohlenstoffatomen und Y für ein Alkyl- undloder Erdalkalimetall, Ammonium, Alkylammonium, Alkanolammonium oder Glucammonium steht. Typische Beispiele für Alkylsulfate, die Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen' schen Oxosynthese erhalten werden. Die Sulfatierungsprodukte können vorzugsweise in Form ihrer Alkalisalze, und insbesondere ihrer Natriumsalze eingesetzt werden. Besonders bevorzugt sind Alkylsulfate auf Basis von C_{16/18}-Talgfettalkoholen bzw. pflanzlicher Fettalkohole vergleichbarer C-Kettenverteilung in Form ihrer Natriumsalze.

### Hilfs- und Zusatzstoffe

Die erfindungsgemäßen Mittel können übliche Hilfs- und Zusatzstoffe, wie beispielsweise Parfümöle, Farbstoffe und Konservierungsmittel enthalten. Vorzugsweise kommen als Zusatzstoffe **schmutzablösende Polymere** in Betracht. Bei diesen sogenannten "soil repellants" handelt es sich um Polymere, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt vorzugsweise im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease®T (ICI) oder Repelotex® SRP 3 (Rhône-Poulenc). Ferner kommen auch sulfonierte Typen der BASF in Frage. Der Anteil weiterer Tenside, Hilfs- undloder Zusatzstoffe kann - bezogen auf den Feststoffgehalt der Mittel 1 bis 10, vorzugsweise 4 bis 8 Gew.-% ausmachen.

### Gewerbliche Anwendbarkeit

Die erfindungsgemäßen Mittel sind lagerstabil und weisen eine vorteilhafte Viskosität auf. Sie eignen sich zur Avivage von Fasern, Garnen und textilen Flächengebilden im allgemeinen und Wäsche im besonderen. Ein weiterer vorteilhafter Effekt besteht in einer antistatischen Ausrüstung der genannten Textilien durch die erfindungsgemäßen Mittel. Es ist ferner möglich, die beobachteten Effekte auch im Bereich von Keratinfasern, d.h. in der Haarkosmetik zu nutzen. Ein weiterer Gegenstand der Erfindung betrifft die Verwendung von Polyolefinwachsen als Verdickungsmittel für wäßrige Esterquatlösungen.

### Beispiele

**Beispiel 1.** In einem 2-l-Becherglas wurden 450 ml Wasser vor- gelegt, auf 35°C erhitzt und unter Rühren zunächst mit 20 g einer pseudokationischen Polyethylenwachsdispersion (Adalin® K, Henkel KGaA, Düsseldorf/FRG, pH = 5, spezifisches Gewicht 1 g/cm³) und dann mit 50 g methylquaterniertem Dipalmfettsäuretriethanolaminester-Methylsulfatsalz (Dehyquart® AU46, Pulcra S.A., Barcelona/ES) versetzt. Danach wurden 3 ml Parfümöl und 2 ml Formalinlösung zugegeben und die Mischung mit Wasser (20°C) bis auf ein Volumen von 1 verdünnt. Die resultierenden Dispersionen wurden drei Wochen bei 5, 20 und 40°C gelagert nach die Viskosität im Abstand von einer Woche nach der Brookfieldmethode bestimmt (Spindel 1, 20 Upm). Die Ergebnisse sind in Tabelle 1 zusammengefaßt.

**Vergleichsbeispiel 1.** Beispiel 1 wurde wiederholt, die pseudokationische Polyethylenwachsdispersion jedoch nach dem Esterquat zudosiert. Die Ergebnisse sind in Tabelle 1 zusammengefaßt.

**Vergleichsbeispiel 2.** Beispiel 1 wurde wiederholt, Parfümöl und Formalin jedoch erst zum Schluß zu der verdünnten Lösung gegeben. Die Ergebnisse sind in Tabelle 1 zusammengefaßt.

**Vergleichsbeispiel 3.** Beispiel 1 wurde wiederholt, auf die Zugabe der pseudokationischen Polyethylenwachsdispersion jedoch verzichtet. Die Ergebnisse sind in Tabelle 1 zusammengefaßt.

**Tabelle 1**

| **Viskosität und Lagerbeständigkeit** | | | | | | | | |
|---|---|---|---|---|---|---|---|---|
| **Beispiele** | **Viskosität [mPas]** | | | | | | | |
| | **sofort** | **nach 1 Woche** | | | **nach 2 Wochen** | | **nach 3 Wochen** | |
| | 20°C | 5°C | 20°C | 40°C | 20°C | 40°C | 20°C | 40°C |
| 1 | 112 | 106 | 112 | 110 | 116 | 110 | 120 | 108 |
| V1 | 20 | 20 | 20 | 22 | 24 | 22 | 24 | 22 |
| V2 | 26 | 26 | 26 | 26 | 26 | 30 | 26 | 32 |
| V3 | 26 | 28 | 30 | 28 | 28 | 30 | 28 | 30 |

## Patentansprüche

1. Wäßrige Weichspüldispersion auf Basis kationischer Tenside und polymerer Wachse, dadurch erhältlich, daß man
(a) Polyolefinwachse in Wasser einrührt,
(b) Esterquats vom Typ der quaternierten Fettsäurealkanolaminestersalze sowie gegebenenfalls weitere Tenside und Hilfs- undloder Zusatzstoffe zugibt und
(c) die Mischung auf Anwendungskonzentration verdünnt.

2. Verfahren zur Herstellung von wäßrigen Weichspülerdispersionen, bei dem man
(a) Polyolefinwachse in Wasser einrührt,
(b) Esterquats vom Typ der quaternierten Fettsäurealkanolaminestersalze sowie gegebenenfalls weitere Tenside und Hilfs- und/oder Zusatzstoffe zugibt und
(c) die Mischung auf die Anwendungskonzentration verdünnt.

3. Verfahren nach Anspruch 2, **dadurch gekennzeichnet,** daß man Esterquats der Formel **(I)** einsetzt, 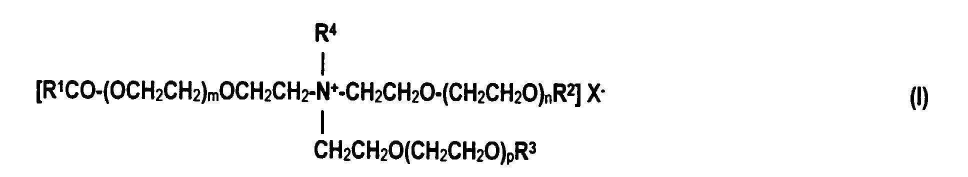 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

4. Verfahren nach Anspruch 2, **dadurch gekennzeichnet,** daß man Esterquats der Formel **(II)** einsetzt, 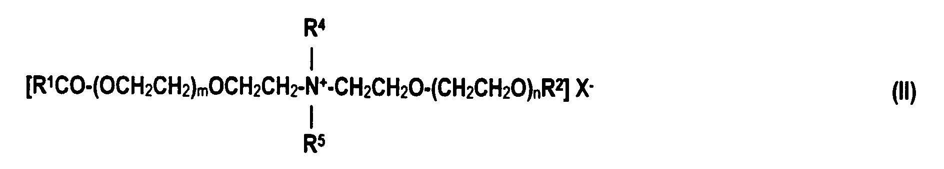 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verfahren nach Anspruch 2, **dadurch gekennzeichnet**, daß man Esterquats der Formel **(III)** einsetzt, 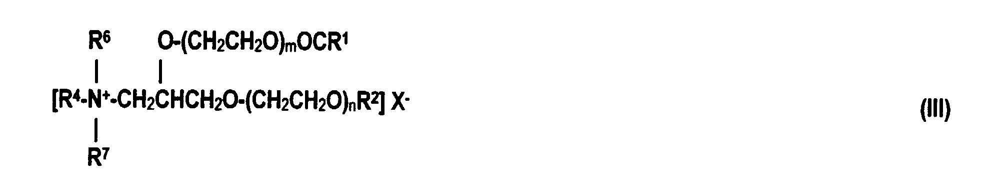 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

6. Verfahren nach den Ansprüchen 2 bis 5, **dadurch gekennzeichnet,** daß man als Polyolefinwachse pseudokationische high-density Polyethylenwachse mit einem mittleren Molekulargewicht im Bereich von 500 bis 10.000 einsetzt.

7. Verfahren nach den Ansprüchen 2 bis 6, **dadurch gekennzeichnet**, daß man die Esterquats und Polyolefinwachse - jeweils bezogen auf ihren Feststoffanteil - im Gewichtsverhältnis von 90 : 10 bis 50 : 50 einsetzt.

8. Verfahren nach den Ansprüchen 2 bis 7, **dadurch gekennzeichnet,** daß man die wäßrigen Lösungen enthaltend die Esterquats, Polyolefinwachse und gegebenenfalls weitere Tenside, Hilfs- und Zusatzstoffe auf eine Anwendungskonzentration von 1 bis 10 Gew.-% Feststoff verdünnt.

9. Verfahren nach den Ansprüchen 2 bis 8, **dadurch gekennzeichnet**, daß man als weitere Tenside Alkyl- und/oder Alkenylsulfate der Formel **(IV)** einsetzt,
**R**^{**8**}**O-SO**_{**3**}**Y** (IV)
in der R⁸ für einen Alkyl- und/oder Alkenylrest mit 8 bis 22 Kohlenstoffatomen und Y für ein Alkali- und/oder Erdalkalimetall, Ammonium, Alkylammonium, Alkanolammonium oder Glucammonium steht.

10. Verwendung von Polyolefinwachsen als Verdickungsmittel für wäßrige Esterquatlösungen.

## Claims

1. An aqueous fabric softener dispersion based on cationic surfactants and polymeric waxes obtainable by
(a) stirring polyolefin waxes into water,
(b) adding esterquats of the quaternized fatty acid alkanolamine ester salt type and optionally other surfactants and auxiliaries and/or additives and
(c) diluting the mixture to the in-use concentration.

2. A process for the production of aqueous fabric softener dispersions in which
(a) polyolefin waxes are stirred into water,
(b) esterquats of the quaternized fatty acid alkanolamine ester salt type and optionally other surfactants and auxiliaries and/or additives are added and
(c) the mixture is diluted to the in-use concentratiion.

3. A process as claimed in claim 2, characterized in that esterquats corresponding to formula (I): 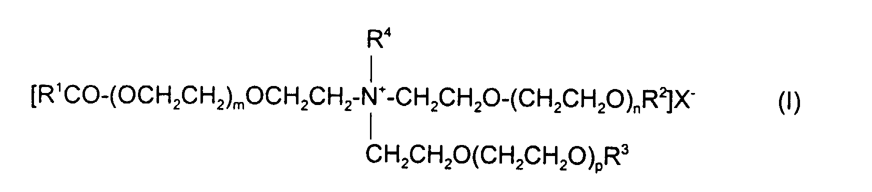 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

4. A process as claimed in claim 2, characterized in that esterquats corresponding to formula (II): 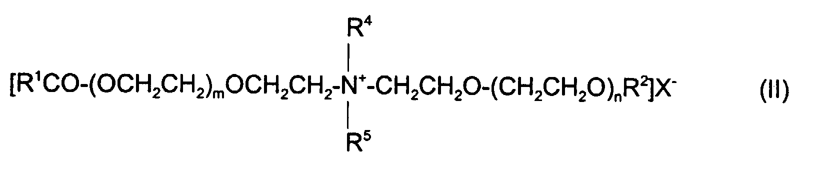 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate,
are used.

5. A process as claimed in claim 2, characterized in that esterquats corresponding to formula (III): 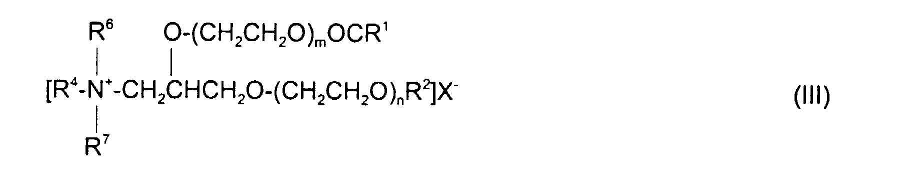 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate,
are used.

6. A process as claimed in claims 2 to 5, characterized in that pseudocationic high-density polyethylene waxes with an average molecular weight of 500 to 10,000 are used.

7. A process as claimed in claims 2 to 6, characterized in that the esterquats and polyolefin waxes are used in a ratio by weight of 90:10 to 50:50, based on the respective solids contents.

8. A process as claimed in claims 2 to 7, characterized in that the aqueous solutions containing the esterquats, polyolefin waxes and optionally other surfactants, auxiliaries and additives are diluted to an in-use concentration of 1 to 10% by weight solids.

9. A process as claimed in claims 2 to 8, characterized in that alkyl and/or alkenyl sulfates corresponding to formula (IV):
R⁸-SO₃Y (IV)
in which R⁸ is an alkyl and/or alkenyl group containing 8 to 22 carbon atoms and Y is an alkali metal and/or alkaline earth metal, ammonium, alkylammonium, alkanolammonium or glucammonium.

10. The use of polyolefin waxes as thickeners for aqueous esterquat solutions.

## Revendications

1. Dispersion aqueuse d'agents assouplissants à base d'agents tensioactifs cationiques et de cires polymères que l'on peut obtenir
(a) en délayant des cires de polyoléfine dans l'eau,
(b) en ajoutant des esterquats du type des sels d'ester d'alcanolamine d'acides gras quaternisés ainsi que le cas échéant d'autres agents tensioactifs et adjuvants et/ou additifs et
(c) en diluant le mélange à la concentration d'application.

2. Procédé de production de dispersions aqueuses d'agents assouplissants dans lequel,
(a) on délaye des cires de polyoléfine dans l'eau,
(b) on ajoute des esterquats du type des sels d'ester d'alcanolamine d'acides gras quaternisés ainsi que le cas échéant d'autres agents tensioactifs et adjuvants et/ou additifs et
(c) on dilue le mélange à la concentration d'application.

3. Procédé selon la revendication 2,
caractérisé en ce qu'
on utilise des esterquats de formule (I), 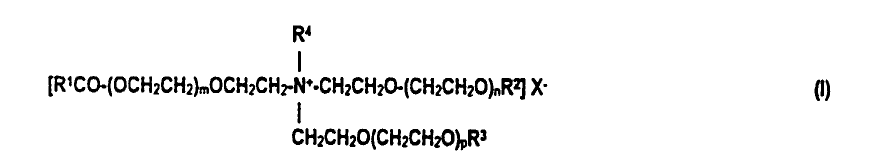 dans laquelle R¹CO représente un radical acyle ayant de 6 à 22 atomes de carbone, R² et R³ représentent indépendamment l'un de l'autre un hydrogène ou R¹CO, R⁴ représente un radical alkyle ayant de 1 à 4 atomes de carbone ou un groupe (CH₂CH₂O)_{q}H, m, n et p valent au total 0 ou des nombres allant de 1 à 12, q représente des nombres compris entre 1 et 12 et X représente un halogénure, un sulfate d'alkyle ou un phosphate d'alkyle.

4. Procédé selon la revendication 2,
caractérisé en ce qu'
on utilise des esterquats de formule (II), 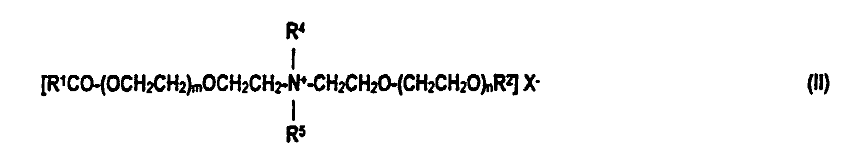 dans laquelle R¹CO représente un radical acyle ayant de 6 à 22 atomes de carbone, R² et R³ représente un hydrogène ou R¹CO, R⁴ et R⁵ représentent indépendamment l'un de l'autre des radicaux alkyle ayant de 1 à 4 atomes de carbone, m et n valent au total 0 ou des nombres de 1 à 12 et X représente un halogénure, un sulfate d'alkyle ou un phosphate d'alkyle.

5. Procédé selon la revendication 2,
caractérisé en ce qu'
on utilise des esterquats de formule (III), 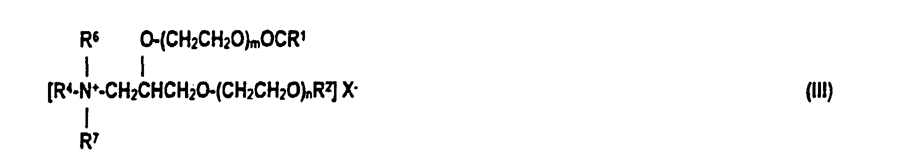 dans laquelle R¹CO représente un radical acyle ayant de 6 à 22 atomes de carbone, R² représente un hydrogène ou R¹CO, R⁴, R⁶ et R⁷ représentent indépendamment l'un de l'autre des radicaux alkyle ayant de 1 à 4 atomes de carbone, m et n valent au total 0 ou des nombres de 1 à 12 et X représente un halogénure, un sulfate d'alkyle ou un phosphate d'alkyle.

6. Procédé selon les revendications 2 à 5,
caractérisé en ce qu'
on utilise comme cires de polyoléfine des cires de polyéthylène haute densité pseudocationiques ayant un poids moléculaire moyen compris entre 500 et 10 000.

7. Procédé selon les revendications 2 à 6,
caractérisé en ce qu'
on utilise les esterquats et les cires de polyoléfine - toujours par rapport à leur teneur en solides - dans un rapport pondéral de 90:10 à 50::50.

8. Procédé selon les revendications 2 à 7,
caractérisé en ce qu'
on dilue les solutions aqueuses contenant les esterquats, les cires de polyoléfine et le cas échéant d'autres agents tensioactifs, adjuvants et additifs à une concentration d'application de 1 à 10 % en poids de solides.

9. Procédé selon les revendications 2 à 8,
caractérisé en ce qu'
on utilise comme autres agents tensioactifs des sulfates d'alkyle et/ou d'alcényle de formule (IV),
**R**^{**8**}**O-SO**_{**3**}**Y** (IV)
dans laquelle R⁸ représente un radical alkyle et/ou alcényle ayant de 8 à 22 atomes de carbone, et Y représente un métal alcalin et/ou alcalino-terreux, l'ammonium, un alkylammonium, un alcanolammonium ou glucammonium.

10. Utilisation de cires de polyoléfines comme agents épaississants pour solutions aqueuses d'esterquats.
